# EUROPEAN PATENT APPLICATION

(11) **EP 1 570 820 A1**
(43) Date of publication of application: **07.09.2005**
(21) Application number: 04077822.7
(22) Date of filing: 14.10.2004
(51) Int. Cl.: A61F 5/453

(54) **Support device for external catheter**

(30) Priority: 01.03.2004 PL 36572704
(71) Applicant: Zurek, Aleksy, 32-415 Czaslaw (PL); Dobrowolski, Zygmunt, 31-106 Krakow (PL)
(72) Inventor: Zurek, Aleksy, 32-415 Czaslaw (PL); Dobrowolski, Zygmunt, 31-106 Krakow (PL)
(74) Representative: Bartula-Toch, Marta

(57) **Abstract**

The present solution consists of a flap component (1) with aperture (2) of dimensions comparable to those of the catheter body (5) and considerably smaller than those of the cap (6) of the catheter (5). The flap component (1) has fastening elements (3) to fix the catheter to the patient's body. These elements are in the form of extended lugs of shape resembling that of shorts, or straps, also flexible. The fastening elements (3) are easily fixed permanently or temporarily to the flap component (1) with fasteners (4) being a hole, button, lace eye, clasp, Velcro, clamp or snap.

## Description

The object of the present invention is a fastening element that fixes a cap catheter for urine drainage to be utilized by men who due to various diseases, injuries and surgical interventions have no voluntary control over urination and thus urinating continuously.

Currently, internal catheters introduced into the urethra are commonly used in hospitals. These devices, however, may cause infections that can be carried from the urethra into the bladder and kidney. Such infections are difficult to control and dangerous for elder men who often suffer from other diseases too. In addition, introducing a catheter is difficult, time-consuming and uncomfortable.

Another common solution is an external catheter disposed about the penis. Such device takes the form of a condom that slides out from the penis when the patient is urinating or moving, thus making that solution unusable. To clear such defect the catheter is fixed to the penis or its surrounding areas with a glue or adhesive tapes, thus causing skin irritation, chafes and wounds.

The set for incontinent men is known form the Polish patent No. 42016. The set is provided with loin-cloth placed on the felt prop, joined with buttons and one of the four tabs of the disk. The disk has perpendicular muff with a groove. A condom is put on the muff and it is fixed to the groove with a ring. The condom is joined with urine discharge tubing coupled to an urine reservoir.

The present invention comprise a flexible flap component with aperture of dimensions comparable to those of the catheter body, understood as its short section for elbow shaped catheters or short ring around the catheter side hole, or slot clearance between the bottom of the cap and the catheter. At the same time the flap component aperture is considerably smaller than the catheter cap. The flap component has fastening elements to be fixed to the patient's body in the form of extended lugs resembling shorts in shape and comfortably fastened to the body, while remaining free ends in the buttock or/and hip areas, or in the form of straps, also flexible, permanently or temporarily coupled to them with fasteners. The fasteners shall be a hole, button, lace eye, clasp, Velcro, clamp or snap.
Advantageously, the flap component is of rectangular or inverted trapezium shape with straps fixed to its corners.
The free ends of straps of the fastening elements are tied or coupled with fasteners, advantageously in the form of a hole, button, lace eye, clasp, Velcro, clamp or snap.

Advantageously, the fastening element takes the form of adjustable suspenders worn around the waist, whereas the flap component and/or expanded surface fastening elements are perforated.

The main advantage of the present invention presented above is a new method of the catheter fastening to the body avoiding mechanical loads to the penis as well as chafes and allergic skin reactions.

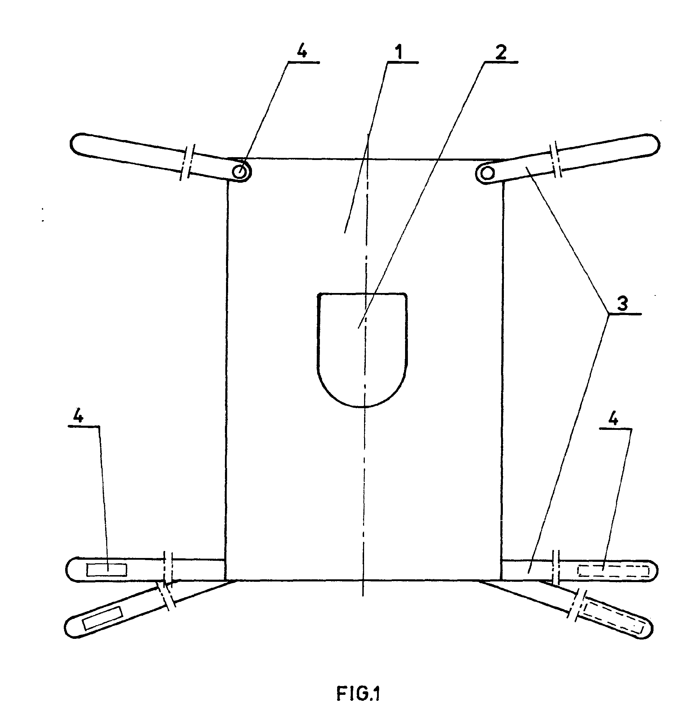

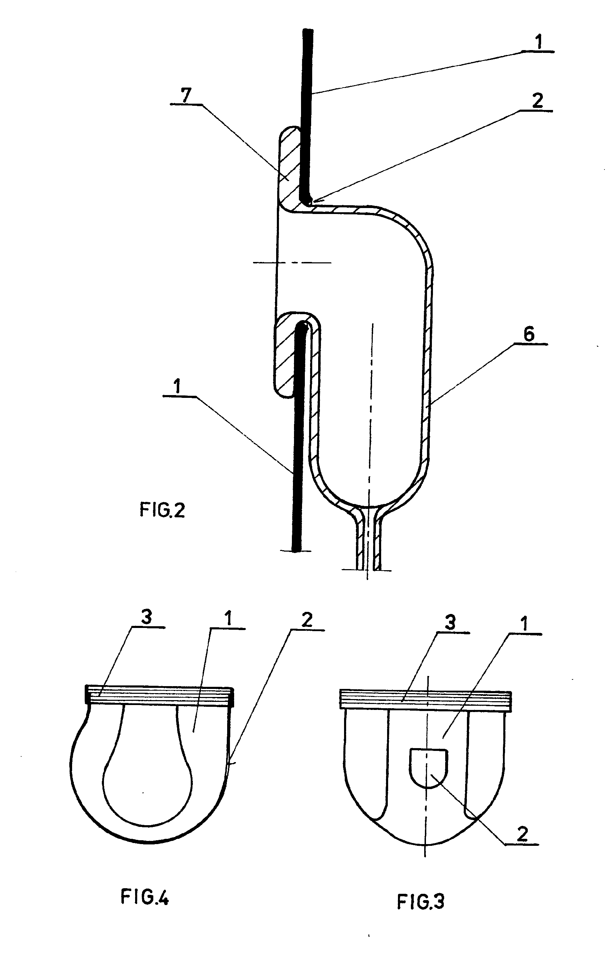

An example of embodiment of the invention is presented in Fig 1 showing front view of the flap component 1 in the form of flexible rectangular flap. Fig. 2 presents longitudinal cross-section of the catheter 6 complete with disposed flap components 1. Fig. 3 : front view of the flap component 1 of shorts-like shape, Fig. 4 - side view of the flap component 1 of shorts-like shape.

### Example I

The fastening elements is a flexible flap component 1 of rectangular shape and placed vertically. In the flap component 1 there is an aperture 2 of dimensions slightly larger than those of 90° open elbow section of the catheter 6 and considerably smaller than those of oval cap 7 that ends the short bent section of the catheter 6.

In the corners of the flap component 1 there are fastening elements 3 joined in various ways, whereas two upper elements are sewn, two lower left ones are coupled with Velcro fasteners 4 and two lower right ones are joined with fasteners 4 in the form of holes and buttons sewn to the flap component 1. The upper left fastening element 3 is ended with a fastener 4 in the form of clasp, while the upper right fastening element 3 has a raw of small holes enabling clasp joining with the upper left fastening element 3.

The flap component 1 is disposed onto the catheter 6 by passing the flexible cap 7 through the aperture 2. The flap component 1 is fixed to the patient's body, by stretching the fastening elements 3 around the waist and clamping with fasteners 4 in the form of clasp. The lower fastening elements 4 are tied around the patient's thighs. The fastened flap component 1 rests on the cap 7 of the catheter 6 around the aperture 2, thus coupling it temporarily to the underbelly.

### Example II

The flap component 1 is of elongated rectangular shape with the aperture 2. The lower part of the flap component 1 is sewn to the fastening element 3 in the form of triangle shaped flap. The upper sides of the flap component 1 and the fastening element 3 are sewn to a rubber strap twisted around the waist.

The flap component 1 is disposed onto the catheter 6 in the same way as described above for Example I.

## Claims

**1.** A fastening element fixing the cap catheter of the urine drainage system, wherein the fastening element has a flexible flap component (1) with aperture (2) of dimensions comparable to those of the catheter body (5) and considerably smaller than the cap (6) of the catheter (5), and has fastening elements (3) to be fixed to the patient's body in the form of extended shorts-like shaped lugs, comfortably fastened to the body, while remaining free ends in the buttock or/and hip areas, or in the form of straps, also flexible, permanently or temporarily coupled to them with fasteners (4). The fasteners shall be a hole, a button, a lace eye, a clasp, Velcro, a clamp or snap.

**2.** The element of claim 1, wherein the flap component (1) is of rectangular-like or inverted trapezium shape.

**3.** The element of claim 2, wherein the strap fastening elements (3) are fixed to the corners of the flap component (1).

**5.** The element of claim 1, wherein the free ends of the strap fastening elements (3) are tied or joined with fastenings (4), advantageously in the form of a hole, a button, a lace eye, a clasp, Velcro, a clamp or snap.
